# EUROPÄISCHE PATENTSCHRIFT

(11) **EP 0 814 077 B1**
(45) Veröffentlichungstag und Bekanntmachung des Hinweises auf die Patenterteilung: **05.04.2000**
(21) Anmeldenummer: 97109546.8
(22) Anmeldetag: 12.06.1997
(51) Int. Cl.: C07C 317/42, C07C 311/47, A61K 31/10, A61K 31/18

(54) **Benzoylguanidine, Verfahren zu ihrer Herstellung sowie sie enthaltendes Medikament**
Benzoylguanidines, process for their preparation and medicaments containing them
Benzoylguanidines, procédé pour leur préparation et medicaments les contenant

(30) Priorität: 18.06.1996 DE 19624178
(43) Veröffentlichungstag der Anmeldung: 29.12.1997
(73) Patentinhaber: HOECHST AKTIENGESELLSCHAFT, 65929 Frankfurt am Main (DE)
(72) Erfinder: Weichert, Andreas, Dr., 63329 Egelsbach (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Schwark, Jan-Robert, Dr., 65929 Frankfurt (DE); Albus, Udo, Dr., 61197 Florstadt (DE); Scholz, Wolfgang, Dr., 65760 Eschborn (DE)

(56) Entgegenhaltungen:
- EP-A- 0 628 543
- EP-A- 0 699 663
- EP-A- 0 699 666

## Beschreibung

Die Erfindung betrifft Benzoylguanidine der Formel I 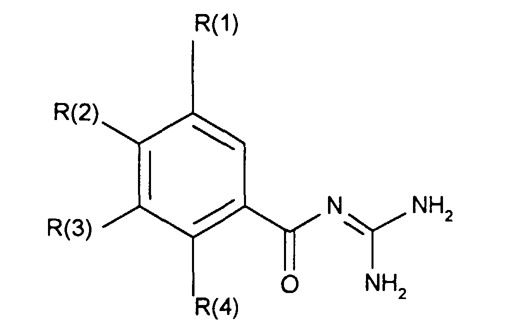 worin bedeuten:
R(1) R(13)-SOₘ oder R(14)R(15)N-SO₂-;
m 1 oder 2;
R(13) Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(16),
n Null, 1, 2, 3 oder 4;
R(16) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(25)R(26);
R(25) und R(26)
unabhängig voneinander Wasserstoff, Alkyl mit 1, 2, 3 oder 4 C-Atomen oder Perfluoralkyl mit 1, 2, 3 oder 4 C-Atomen;
R(14) Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(27),
n Null, 1, 2, 3 oder 4;
R(27) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(28)R(29);
R(28) und R(29)
unabhängig voneinander Wasserstoff, Alkyl mit 1, 2, 3 oder 4 C-Atomen oder Perfluoralkyl mit 1, 2, 3 oder 4 C-Atomen;
R(15) Wasserstoff, Alkyl mit 1, 2, 3 oder 4 C-Atomen oder Perfluoralkyl mit 1, 2, 3 oder 4 C-Atomen;
oder
R(14) und R(15)
gemeinsam 4 oder 5 Methylengruppen, von denen eine CH₂-Gruppe durch Sauerstoff, S, NH, N-CH₃ oder N-Benzyl ersetzt sein kann;
einer der Substituenten R(2) und R(3)
Wasserstoff;
und der jeweils andere Substituent R(2) und R(3)
-CHR(30)R(31);
R(30)
-(CH₂)_{g}-(CHOH)ₕ-(CH₂)ᵢ-(CHOH)ₖ-R(32) oder
-(CH₂)_{g}-O-(CH₂-CH₂O)ₕ-R(24);
R(24 und R(32)
unabhängig voneinander Wasserstoff oder Methyl;
g, h, i
gleich oder verschieden Null, 1, 2, 3 oder 4;
k 1, 2, 3 oder 4;
oder der jeweils andere Substituent R(2) und R(3)
-C(OH)R(33)R(34);
R(31), R(33) und R(34)
gleich oder verschieden Wasserstoff oder Alkyl mit 1, 2, 3 oder 4 C-Atomen
oder
R(33) und R(34)
gemeinsam Cycloalkyl mit 3, 4, 5 oder 6 C-Atomen;
oder
R(33) -CH₂OH;
R(4) Alkyl mit 1, 2, 3 oder 4 C-Atomen, Alkoxy mit 1, 2, 3 oder 4 C-Atomen, F, Cl, Br, I, CN, -(CH₂)ₙ-(CF₂)ₒ-CF₃;
n Null oder 1;
o Null, 1 oder 2;
sowie deren pharmazeutisch verträgliche Salze.

Bevorzugt sind Verbindungen der Formel I, in denen bedeuten:
R(1) R(13)-SO₂ oder R(14)R(15)N-SO₂-;
R(13) Alkyl mit 1, 2, 3 oder 4 C-Atomen, Perfluoralkyl mit 1, 2, 3 oder 4 C-Atomen, Alkenyl mit 3 oder 4 C-Atomen oder -CₙH₂ₙ-R(16),
n Null, 1, 2, 3 oder 4;
R(16) Cycloalkyl mit 3, 4, 5 oder 6 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl oder Methoxy;
R(14) Wasserstoff, Alkyl mit 1, 2, 3 oder 4 C-Atomen, Perfluoralkyl mit 1, 2, 3 oder 4 C-Atomen, Alkenyl mit 3 oder 4 C-Atomen oder -CₙH₂ₙ-R(27),
n Null, 1, 2, 3 oder 4;
R(27) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl oder Methoxy;
R(15) Wasserstoff, Alkyl mit 1, 2, 3 oder 4 C-Atomen oder Perfluoralkyl mit 1, 2, 3 oder 4 C-Atomen;
oder
R(14) und R(15)
gemeinsam 4 oder 5 Methylengruppen, von denen eine CH₂-Gruppe durch Sauerstoff, S, NH, N-CH₃ oder N-Benzyl ersetzt sein kann;
einer der Substituenten R(2) und R(3)
Wasserstoff;
und der jeweils andere Substituent R(2) und R(3)
-CHR(30)R(31);
R(30)
-(CH₂)_{g}-(CHOH)ₕ-(CH₂)ᵢ-(CHOH)ₖ-R(32) oder
-(CH₂)_{g}-O-(CH₂-CH₂O)ₕ-R(24);
R(24) und R(32)
unabhängig voneinander Wasserstoff oder Methyl;
g, h, i
gleich oder verschieden Null, 1 oder 2;
k 1 oder 2;
oder
der jeweils andere Substituent R(2) und R(3)
-C(OH)R(33)R(34);
R(33) und R(34)
gleich oder verschieden Wasserstoff oder Alkyl mit 1, 2, 3 oder 4 C-Atomen
oder
R(33) und R(34)
gemeinsam Cycloalkyl mit 3, 4, 5 oder 6 C-Atomen;
oder
R(33) -CH₂OH;
R(4) Alkyl mit 1, 2, 3 oder 4 C-Atomen, Alkoxy mit 1, 2, 3 oder 4 C-Atomen, F, Cl, CN oder (CF₂)ₒ-CF₃;
o Null, 1 oder 2;
sowie deren pharmazeutisch verträgliche Salze.

Ganz besonders bevorzugt sind Verbindungen der Formel I, in denen bedeuten:
R(1) R(13)-SO₂;
R(13) Alkyl mit 1, 2, 3 oder 4 C-Atomen;
einer der Substituenten R(2) und R(3)
Wasserstoff;
und der jeweils andere Substituent R(2) und R(3)
-C(OH)(CH₃)-CH₂OH, -CH(CH₃)-CH₂OH oder C(OH)(CH₃)₂;
R(4) Alkyl mit 1, 2, 3 oder 4 C-Atomen, Alkoxy mit 1, 2, 3 oder 4 C-Atomen, F, Cl, CN oder -CF₃;
sowie deren pharmazeutisch verträgliche Salze.

Unter Heteroaryl mit 1, 2, 3, 4, 5, 6, 7, 8 oder 9 C-Atomen werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein. Als Heteroaryl mit 1, 2, 3, 4, 5, 6, 7, 8 oder 9 C-Atomen gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(4) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft außerdem ein Verfahren zur Herstellung einer Verbindung der Formel I, das dadurch gekennzeichnet ist, daß man
eine Verbindung der Formel II 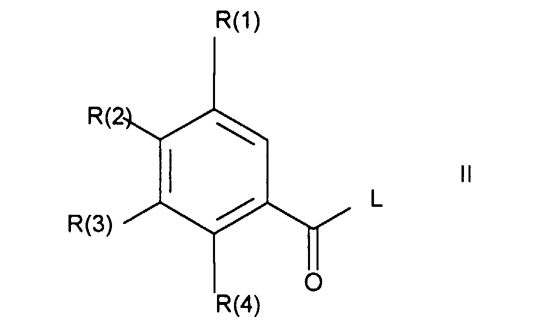 worin R(1) bis R(4) die angegebene Bedeutung besitzen und L für eine leicht nucleophil substituierbare Fluchtgruppe steht,
mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-Derivaten (Formel II, L = OH) herstellen, wie die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel. Die Aktivierung von Benzoesäuren kann auch mit mit Dicyclohexylcarbodiimid (DCC) oder mit O-[(Cyano(ethoxycarbonyl)methylen)amino]-1,1,3,3-tetramethyluroniumtetrafluorborat ("TOTU") erfolgen [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 2-, 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Aus der EP-OS 640 588 A 1 sind Benzoylguanidine ähnlicher Struktur bekannt, die jedoch keine Alkylsulfonyl- oder Alkylsulfamoylgruppe aufweisen, welche die erfindungsgemäßen Verbindungen stets aufweisen.

Die EP-OS 699 660 betrifft auch ortho-substituierte Benzoylguanidine, die jedoch nicht die erfindungsgemäße Hydroxylgruppe im Substituenten R(2) oder R(3) aufweisen.

Die ältere deutsche Anmeldung 196 08 162.9 (HOE 96/F 042) schlägt auch orthosubstituierte Benzoylguanidine vor, die aber in keinem Falle eine Alkylsulfonylgruppe enthalten.

Gegenüber den bekannten Verbindungen zeichnen sich die erfindungsgemäßen Verbindungen durch eine hohe Wirksamkeit in der Inhibition des Na⁺/H⁺-Austauschs, kombiniert mit einer sehr guten Wasserlöslichkeit, aus.

Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheiten wichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch schäme ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und - hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, daß Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, daß Verbindungen der Formel I bezüglich der Beeinflussung der Serumlipidspiegel wertvolle therapeutisch verwertbare Eigenschaften zeigen. So erniedrigen sie signifikant die erhöhten Serum Konzentration von LDL und VLDL, wie sie beispielweise durch erhöhte dietätische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einem wirksamen Schutz gegen Schädigungen des Endothels, die durch Stoffwechselanomalien hervorgerufen werden. Mit diesem Schutz der Gefäße gegen das Syndrom der endothelialen Dysfunktion sind Verbindungen der Formel I wertvolle Arzneimittel zur Prävention und zur Behandlung koronarer Gefäßsspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atheroskleroseinduzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq.: Äquivalent

### Experimenteller Teil

### Allgemeine Vorschrift zur Herstellung von Benzoyl-guanidinen (I) Variante A: aus Benzoesäuren (II, L=OH)

1.0 eq. des Benzoesäurederivates der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Sieden erhitzt (typische Reaktionszeit 2 bis 5 h). Das Lösungsmittel wird unter vermindertem Druck (Rotationsverdampfer) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.

### (Salzbildung vergleiche Variante A)

Beispiel 1: 2-Chlor-3-(1'-hydroxy-2'-propyl)-5-methylsulfonyl-benzoylguanidin Farblose Kristalle,
Fp. 211°C (dec.).

### Syntheseweg:

a) 2-Chlor-3-iodo-5-methylsulfonyl-benzoesäuremethylester aus 2-Chlor-5-methylsulfonyl-benzoesäuremethylester durch Olah-Iodierung mit 1 eq N-Iod-Succinimid in 5 eq Trifluormethansulfonsäure bei RT für 24 h, farblose Kristalle,
   Fp. 155 - 58°C.
b) 2-Chlor-3-isopropenyl-5-methylsulfonyl-benzoesäuremethylester aus 2-Chlor-3-iodo-5-methylsulfonyl-benzoesäuremethylester durch cross-coupling mit 1.5 Äquivalenten Isopropenylzinkchlorid in THF unter Rückfluß in Gegenwart von katalytischen Mengen Palladium(II)acetat und Kupfer(I)iodid, wäßrige Aufarbeitung, Extraktion mit Essigester und anschließende Säulenchromatographie an Kieselgel mit Essigester/n-Heptan (3 : 7).
   farbloses Öl, MS: M⁺ + H = 289.
c) 2-Chlor-3-[1'-hydroxy-2'-propyl]-5-methylsulfonyl-benzoesäure durch Hydro-borierung von 2-Chlor-3-isopropenyl-5-methylsulfonylbenzoesäuremethylester mit Boran-THF-Komplex in THF unter Rückfluß für 3 h, farblose Kristalle,
   Fp. 175 - 77°C.
d) 2-Chlor-3-[1'-hydroxy-2'-propyl]-5-methylsulfonyl-benzoesäuremethylester aus c) durch Veresterung mit 3 eq Methyliodid in Gegenwart von Kaliumkarbonat in DMF bei RT für 3 h, wäßrige Aufarbeitung, gelbliches Öl, M⁺ + H = 307.
e) 2-Chlor-3-[1'-hydroxy-2'-propyl]-5-methylsulfonyl-benzoylguanidin aus d) nach allgemeiner Vorschrift, Variante B.

Beispiel 2: 2-Methoxy-3-(1'-hydroxy-2'-propyl)-5-methylsulfonyl-benzoylguanidin Farblose Kristalle,
Fp. 179 - 80°C (dec.).

### Syntheseweg:

a) 2-Methoxy-5-methylsulfonyl-benzoesäure aus 2-Chlor-5-methylsulfonyl-benzoesäuremethylester mit 10 eq Natriummethanolat in Gegenwart von Kupfer(II)chlorid in Methanol unter Rückfluß für 4 h,
   gelblicher Feststoff,
   Fp. 190 - 92°C.
b) 2-Methoxy-3-iodo-5-methylsulfonyl-benzoesäure aus 2-Methoxy-5-methylsulfonyl-benzoesäure durch Olah-Iodierung mit 1 eq N-Iod-Succinimid in 5 eq Trifluormethansulfonsäure bei RT für 24 h,
   farblose Kristalle,
   Fp. 205 - 07°C.
c) 2-Methoxy-3-iodo-5-methylsulfonyl-benzoesäuremethylester aus b) durch Veresterung mit Überschuß Chlorwasserstoff in Methanol bei RT für 24 h, wäßrige Aufarbeitung,
   farbloser Feststoff, Fp. 140°C.
d) 2-Methoxy-3-isopropenyl-5-methylsulfonyl-benzoesäuremethylester aus 2-Methoxy-3-iodo-5-methylsulfonyl-benzoesäuremethylester analog Verfahren 1 b),
   farbloses Öl, M⁺ + H = 285.
e) 2-Methoxy-3-[1'-hydroxy-2'-propyl]-5-methylsulfonyl-benzoesäure aus d) analog Verfahren 1 c),
   farbloser Feststoff, amorph, M⁺ + H = 289.
f) 2-Methoxy-3-[1'-hydroxy-2'-propyl]-5-methylsulfonyl-benzoesäuremethylester durch Veresterung mit 3 eq Methyliodid in Gegenwart von Kaliumkarbonat in DMF bei RT für 3 h, wäßrige Aufarbeitung,
   hellgelbes Öl, M⁺ + H = 303.
g) 2-Methoxy-3-[1'-hydroxy-2'-propyl]-5-methylsulfonyl-benzoylguanidin aus 2 f) nach allg. Vorschrift, Variante B.

Beispiel 3: 2-Methyl-3-(1'-hydroxy-2'-propyl)-5-methylsulfonyl-benzoylguanidin Farblose Kristalle,
Fp. 208 - 09°C (dec.).

### Syntheseweg:

a) 2-Methyl-5-methylsulfonyl-benzoesäuremethylester aus 2-Chlor-5-methylsulfonyl-benzoesäuremethylester durch cross-coupling mit 2 eq Methylzinkchlorid in THF / DMF unter Rückfluß in Gegenwart von katalytischen Mengen Palladium(II)acetat, Triphenylphosphin und Kupfer(I)iodid, wäßrige Aufarbeitung, Extraktion mit Essigester und anschließende Säulenchromatographie an Kieselgel mit Essigester/n-Heptan (3 : 7),
   farblose Kristalle,
   Fp. 95 - 96°C.
b) 2-Methyl-3-iodo-5-methylsulfonyl-benzoesäuremethylester aus 2-Methyl-5-methyl-sulfonyl-benzoesäuremethylester durch Olah-Iodierung mit 1 eq N-Iod-Succinimid in 5 eq Trifluormethansulfonsäure bei RT für 24 h,
   farblose Kristalle,
   Fp. 137 - 38°C.
c) 2-Methyl-3-isopropenyl-5-methylsulfonyl-benzoesäuremethylester aus 2-Methyl-3-iodo-5-methylsulfonyl-benzoesäuremethylester durch cross-coupling mit 1.5 Äquivalenten Isopropenylzinkchlorid in THF unter Rückfluß in Gegenwart von katalytischen Mengen Palladium(II)acetat, Triphenylphosphin und Kupfer(I)iodid, wäßrige Autarbeitung, Extraktion mit Essigester und anschließende Säulenchromatographie an Kieselgel mit Essigester/n-Heptan (3 : 7),
   farbloses Öl, M⁺ + H = 269.
d) 2-Methyl-3-[1'-hydroxy-2'-propyl]-5-methylsulfonyl-benzoesäure durch Hydro-borierung von 2-Methyl-3-isopropenyl-5-methylsulfonylbenzoesäuremethylester mit Boran-THF-Komplex in THF unter Rückfluß für 3 h,
   amorpher Feststoff, M⁺ + H = 273. e) 2-Methyl-3-[1'-hydroxy-2'-propyl]-5-methylsulfonyl-benzoesäuremethylester durch Veresterung mit 3 eq Methyliodid in Gegenwart von Kaliumkarbonat in DMF bei RT für 3 h, wäßrige Aufarbeitung,
   hellgelbes Öl, M⁺ + H = 287.
f) 2-Methyl-3-[1'-hydroxy-2'-propyl]-5-methylsulfonyl-benzoylguanidin aus e) nach allgemeiner Vorschrift, Variante B.

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amiloridhemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺-Exchangers:

Die Verbindungen sämtlicher Beispiele weisen IC₅₀-Werte von kleiner als 10 µmolar auf.

## Patentansprüche

1. Benzoylguanidine der Formel I 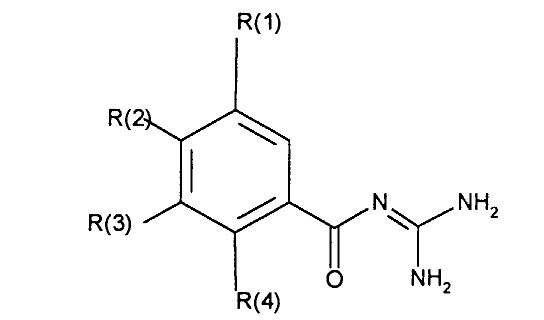 worin bedeuten:
R(1) R(13)-SOₘ oder R(14)R(15)N-SO₂-;
m 1 oder 2;
R(13) Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(16),
n Null, 1, 2, 3 oder 4;
R(16) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(25)R(26);
R(25) und R(26)
unabhängig voneinander Wasserstoff, Alkyl mit 1, 2, 3 oder 4 C-Atomen oder Perfluoralkyl mit 1, 2, 3 oder 4 C-Atomen;
R(14) Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder -CₙH₂ₙ-R(27),
n Null, 1, 2, 3 oder 4;
R(27) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl, Methoxy und NR(28)R(29);
R(28) und R(29)
unabhängig voneinander Wasserstoff, Alkyl mit 1, 2, 3 oder 4 C-Atomen oder Perfluoralkyl mit 1, 2, 3 oder 4 C-Atomen;
R(15) Wasserstoff, Alkyl mit 1, 2, 3 oder 4 C-Atomen oder Perfluoralkyl mit 1, 2, 3 oder 4 C-Atomen;
oder
R(14) und R(15)
gemeinsam 4 oder 5 Methylengruppen, von denen eine CH₂-Gruppe durch Sauerstoff, S, NH, N-CH₃ oder N-Benzyl ersetzt sein kann;
einer der Substituenten R(2) und R(3)
Wasserstoff;
und der jeweils andere Substituent R(2) und R(3)
-CHR(30)R(31);
R(30)
-(CH₂)_{g}-(CHOH)ₕ-(CH₂)ᵢ-(CHOH)ₖ-R(32) oder
-(CH₂)_{g}-O-(CH₂-CH₂O)ₕ-R(24);
R(24 und R(32)
unabhängig voneinander Wasserstoff oder Methyl;
g, h, i
gleich oder verschieden Null, 1, 2, 3 oder 4;
k 1, 2, 3 oder 4;
oder der jeweils andere Substituent R(2) und R(3)
-C(OH)R(33)R(34);
R(31), R(33) und R(34)
gleich oder verschieden Wasserstoff oder Alkyl mit 1, 2, 3 oder 4 C-Atomen
oder
R(33) und R(34)
gemeinsam Cycloalkyl mit 3, 4, 5 oder 6 C-Atomen;
oder
R(33) -CH₂OH;
R(4) Alkyl mit 1, 2, 3 oder 4 C-Atomen, Alkoxy mit 1, 2, 3 oder 4 C-Atomen, F, Cl, Br, I, CN, -(CH₂)ₙ-(CF₂)ₒ-CF₃;
n Null oder 1;
o Null, 1 oder 2;
sowie deren pharmazeutisch verträgliche Salze.

2. Verbindung der Formel I nach Anspruch 1, in der bedeuten:
R(1) R(13)-SO₂ oder R(14)R(15)N-SO₂-;
R(13) Alkyl mit 1, 2, 3 oder 4 C-Atomen, Perfluoralkyl mit 1, 2, 3 oder 4 C-Atomen, Alkenyl mit 3 oder 4 C-Atomen oder -CₙH₂n-R(16),
n Null, 1, 2, 3 oder 4;
R(16) Cycloalkyl mit 3, 4, 5 oder 6 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl oder Methoxy;
R(14) Wasserstoff, Alkyl mit 1, 2, 3 oder 4 C-Atomen, Perfluoralkyl mit 1, 2, 3 oder 4 C-Atomen, Alkenyl mit 3 oder 4 C-Atomen oder -CₙH₂ₙ-R(27),
n Null, 1, 2, 3 oder 4;
R(27) Cycloalkyl mit 3, 4, 5, 6, 7 oder 8 C-Atomen, Phenyl, Biphenylyl oder Naphthyl,
wobei Phenyl, Biphenylyl und Naphthyl nicht substituiert oder substituiert sind mit 1 - 3 Substituenten ausgewählt aus der Gruppe bestehend aus F, Cl, CF₃, Methyl oder Methoxy;
R(15) Wasserstoff, Alkyl mit 1, 2, 3 oder 4 C-Atomen oder Perfluoralkyl mit 1, 2, 3 oder 4 C-Atomen;
oder
R(14) und R(15)
gemeinsam 4 oder 5 Methylengruppen, von denen eine CH₂-Gruppe durch Sauerstoff, S, NH, N-CH₃ oder N-Benzyl ersetzt sein kann;
einer der Substituenten R(2) und R(3)
Wasserstoff;
und der jeweils andere Substituent R(2) und R(3)
-CHR(30)R(31);
R(30)
-(CH₂)_{g}-(CHOH)ₕ-(CH₂)ᵢ-(CHOH)ₖ-R(32) oder
-(CH₂)_{g}-O-(CH₂-CH₂O)ₕ-R(24);
R(24) und R(32)
unabhängig voneinander Wasserstoff oder Methyl;
g, h, i
gleich oder verschieden Null, 1 oder 2;
k 1 oder 2;
oder der jeweils andere Substituent R(2) und R(3)
-C(OH)R(33)R(34);
R(33) und R(34)
gleich oder verschieden Wasserstoff oder Alkyl mit 1, 2, 3 oder 4 C-Atomen;
oder
R(33) und R(34)
gemeinsam Cycloalkyl mit 3, 4, 5 oder 6 C-Atomen;
oder
R(33) -CH₂OH;
R(4) Alkyl mit 1, 2, 3 oder 4 C-Atomen, Alkoxy mit 1, 2, 3 oder 4 C-Atomen, F, Cl, CN oder (CF₂)ₒ-CF₃;
o Null, 1 oder 2.

3. Verbindung der Formel I nach Anspruch 1 oder 2, in der bedeuten:
R(1) R(13)-SO₂;
R(13) Alkyl mit 1, 2, 3 oder 4 C-Atomen;
einer der Substituenten R(2) und R(3)
Wasserstoff;
und der jeweils andere Substituent R(2) und R(3)
-C(OH)(CH₃)-CH₂OH, -CH(CH₃)-CH₂OH oder C(OH)(CH₃)₂;
R(4) Alkyl mit 1, 2, 3 oder 4 C-Atomen, Alkoxy mit 1, 2, 3 oder 4 C-Atomen, F, Cl, CN oder -CF₃.

4. Verfahren zur Herstellung einer Verbindung der Formel I nach Anspruch 1,
dadurch gekennzeichnet, daß man
eine Verbindung der Formel II 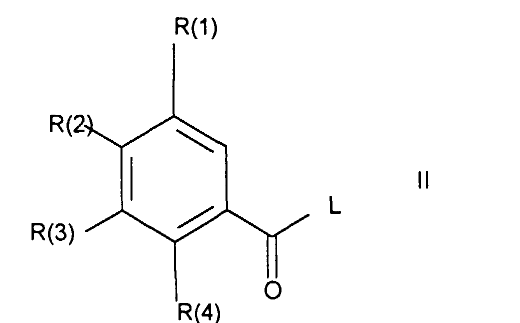 worin R(1) bis R(4) die in Anspruch 1 angegebene Bedeutung besitzen und L für eine leicht nucleophil substituierbare Fluchtgruppe steht,
mit Guanidin umsetzt.

5. Verwendung einer Verbindung I nach Anspruch 1 zur Herstellung eines Medikaments zu Behandlung oder Prophylaxe von durch ischämische Zustände verursachten Krankheiten.

6. Verwendung einer Verbindung I nach Anspruch 1 zur Herstellung eines Medikaments zur Behandlung oder Prophylaxe des Herzinfarkts und von Arrhythmien.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Verwendung einer Verbindung I nach Anspruch 1 zur Herstellung eines wissenschaftliches Tools zur Inhibition des Na⁺/H⁺-Exchangers, zur Diagnose der Hypertonie und proliferativer Erkrankungen.

16. Verwendung einer Verbindung I nach Anspruch 1 zur Herstellung eines Medikaments zur Behandlung oder Prophylaxe von Störungen des Fettstoffwechsels.

17. Arzneimittel, enthaltend eine wirksame Menge einer Verbindung I nach einem oder mehreren der Ansprüche 1 bis 3.

## Claims

1. A benzoylguanidine of the formula I 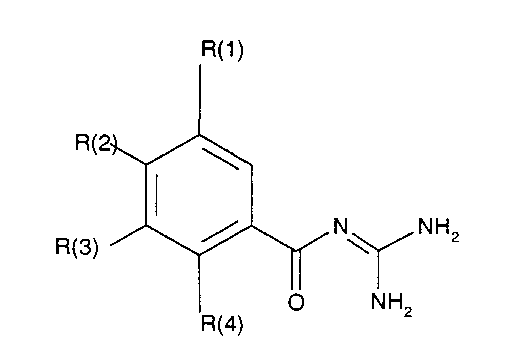 in which:
R(1) is R(13)-SOₘ or R (14)R(15)N-SO₂-;
m is 1 or 2;
R(13) is alkyl having 1, 2, 3, 4, 5, 6, 7 or 8 carbon atoms, perfluoroalkyl having 1, 2, 3, 4, 5, 6, 7 or 8 carbon atoms, alkenyl having 3, 4, 5, 6, 7 or 8 carbon atoms or -CₙH₂ₙ-R(16),
n is zero, 1, 2, 3 or 4;
R(16) is cycloalkyl having 3, 4, 5, 6, 7 or 8 carbon atoms, phenyl, biphenylyl or naphthyl,
where phenyl, biphenylyl and naphthyl are not substituted or are substituted by 1-3 substituents selected from the group consisting of F, Cl, CF₃, methyl, methoxy and NR(25)R(26);
R(25) and R(26) are,
independently of each other, hydrogen, alkyl having 1, 2, 3 or 4 carbon atoms or perfluoroalkyl having 1, 2, 3 or 4 carbon atoms;
R(14) is hydrogen, alkyl having 1, 2, 3, 4, 5, 6, 7 or 8 carbon atoms, perfluoroalkyl having 1, 2, 3, 4, 5, 6, 7 or 8 carbon atoms, alkenyl having 3, 4, 5, 6, 7 or 8 carbon atoms or -CₙH₂ₙ-R(27),
n is zero, 1, 2, 3 or 4;
R(27) is cycloalkyl having 3, 4, 5, 6, 7 or 8 carbon atoms, phenyl, biphenylyl or naphthyl,
where phenyl, biphenylyl and naphthyl are not substituted or are substituted by 1-3 substituents selected from the group consisting of F, Cl, CF₃, methyl, methoxy and NR(28)R(29);
R(28) and R(29) are,
independently of each other, hydrogen, alkyl having 1, 2, 3 or 4 carbon atoms or perfluoroalkyl having 1, 2, 3 or 4 carbon atoms;
R(15) is hydrogen, alkyl having 1, 2, 3 or 4 carbon atoms or perfluoroalkyl having 1, 2, 3 or 4 carbon atoms;
or
R(14) and R(15) are,
together, 4 or 5 methylene groups of which one CH₂ group can be replaced with oxygen, S, NH, N-CH₃ or N-benzyl;
one of the substituents R(2) and R(3) is hydrogen;
and the other of the substituents R(2) and R(3) in each case is
-CHR(30)R(31);
R(30) is
-(CH₂)_{g}-(CHOH)ₕ-(CH₂)ᵢ-(CHOH)ₖ-R(32) or
-(CH₂)_{g}-O-(CH₂-CH₂O)ₕ-R(24);
R(24) and R(32) are,
independently of each other, hydrogen or methyl;
g, h and i are,
identically or differently, zero, 1, 2, 3 or 4;
k is 1, 2, 3 or 4;
or the other of the substituents R(2) and R(3) in each case is
-C(OH)R(33)R(34);
R(31), R(33) and R(34) are,
identically or differently, hydrogen or alkyl having 1, 2, 3 or 4 carbon atoms
or
R(33) and R(34) are,
together, cycloalkyl having 3, 4, 5 or 6 carbon atoms;
or
R(33) is -CH₂OH;
R(4) is alkyl having 1, 2, 3 or 4 carbon atoms, alkoxy having 1, 2, 3 or 4 carbon atoms, F, Cl, Br, I, CN or -(CH₂)ₙ-(CF₂)ₒ-CF₃;
n is zero or 1;
o is zero, 1 or 2;
and the pharmaceutically tolerated salts thereof.

2. A compound of the formula I as claimed in claim 1, in which:
R(1) is R(13)-SO₂ or R (14)R(15)N-SO₂-;
R(13) is alkyl having 1, 2, 3 or 4 carbon atoms, perfluoroalkyl having 1, 2, 3 or 4 carbon atoms, alkenyl having 3 or 4 carbon atoms or -CₙH₂ₙ-R(16),
n is zero, 1, 2, 3 or 4;
R(16) is cycloalkyl having 3, 4, 5 or 6 carbon atoms, phenyl, biphenylyl or naphthyl,
where phenyl, biphenylyl and naphthyl are not substituted or are substituted by 1-3 substituents selected from the group consisting of F, Cl, CF₃, methyl or methoxy;
R(14) is hydrogen, alkyl having 1, 2, 3 or 4 carbon atoms, perfluoroalkyl having 1, 2, 3 or 4 carbon atoms, alkenyl having 3 or 4 carbon atoms or -CₙH₂ₙ-R(27),
n is zero, 1, 2, 3 or 4;
R(27) is cycloalkyl having 3, 4, 5, 6, 7 or 8 carbon atoms, phenyl, biphenylyl or naphthyl,
where phenyl, biphenylyl and naphthyl are not substituted or are substituted by
1-3 substituents selected from the group consisting of F, Cl, CF₃, methyl or methoxy;
R(15) is hydrogen, alkyl having 1, 2, 3 or 4 carbon atoms or perfluoroalkyl having 1, 2, 3 or 4 carbon atoms;
or
R(14) and R(15) are,
together, 4 or 5 methylene groups of which one CH₂ group can be replaced with oxygen, S, NH, N-CH₃ or N-benzyl;
one of the substituents R(2) and R(3) is hydrogen;
and the other of the substituents R(2) and R(3) in each case is -CHR(30)R(31);
R(30) is
-(CH₂)_{g}-(CHOH)ₕ-(CH₂)ᵢ-(CHOH)ₖ-R(32) or
-(CH₂)_{g}-O-(CH₂-CH₂O)ₕ-R(24);
R(24) and R(32) are,
independently of each other, hydrogen or methyl;
g, h and i are,
identically or differently, zero, 1 or 2;
k is 1 or 2;
or the other of the substituents R(2) and R(3) in each case is -C(OH)R(33)R(34);
R(33) and R(34) are,
identically or differently, hydrogen or alkyl having 1, 2, 3 or 4 carbon atoms
or
R(33) and R(34) are,
together, cycloalkyl having 3, 4, 5 or 6 carbon atoms;
or
R(33) is -CH₂OH;
R(4) is alkyl having 1, 2, 3 or 4 carbon atoms, alkoxy having 1, 2, 3 or 4 carbon atoms, F, Cl, CN or (CF₂)_{O}-CF₃;
o is zero, 1 or 2.

3. A compound of the formula I as claimed in claim 1 or 2, in which:
R(1) is R(13)-SO₂;
R(13) is alkyl having 1, 2, 3 or 4 carbon atoms;
one of the substituents R(2) and R(3) is hydrogen;
and the other of the substituents R(2) and R(3) in each case is
-C(OH)(CH₃)-CH₂OH, -CH(CH₃)-CH₂OH or C(OH)(CH₃)₂;
R(4) is alkyl having 1, 2, 3 or 4 carbon atoms, alkoxy having 1, 2, 3 or 4 carbon atoms, F, Cl, CN or -CF₃.

4. A process for preparing a compound of the formula I as claimed in claim 1, which comprises
reacting a compound of the formula II 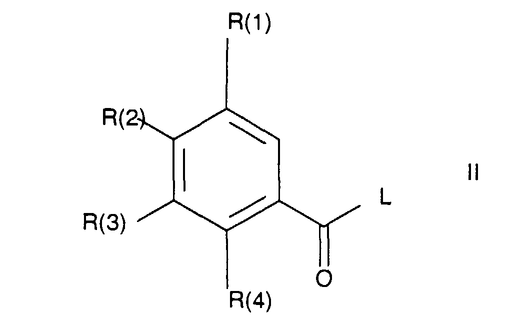 in which R(1) to R(4) have the meaning given in claim 1 and L is a leaving group which can readily be substituted nucleophilically,
with guanidine.

5. The use of a compound I as claimed in claim 1 for preparing a medicament for the treatment or prophylaxis of disorders which are caused by ischemic states.

6. The use of a compound I as claimed in claim 1 for preparing a medicament for the treatment or prophylaxis of cardiac infarction and arrhythmias.

7. The use of a compound I as claimed in claim 1 for preparing a medicament for the treatment or prophylaxis of angina pectoris.

8. The use of a compound I as claimed in claim 1 for preparing a medicament for the treatment or prophylaxis of ischemic states of the heart.

9. The use of a compound I as claimed in claim 1 for preparing a medicament for the treatment or prophylaxis of ischemic states of the peripheral and central nervous system, and of stroke.

10. The use of a compound I as claimed in claim 1 for preparing a medicament for the treatment or prophylaxis of ischemic states of peripheral organs and limbs.

11. The use of a compound I as claimed in claim 1 for preparing a medicament for the treatment of states of shock.

12. The use of a compound I as claimed in claim 1 for preparing a medicament for use in surgical operations and organ transplantations.

13. The use of a compound I as claimed in claim 1 for preparing a medicament for the preservation and storage of transplants for surgical procedures.

14. The use of a compound I as claimed in claim 1 for preparing a medicament for the treatment of disorders in which cell proliferation constitutes a primary or secondary cause.

15. The use of a compound I as claimed in claim 1 for preparing a scientific tool for inhibiting the Na⁺/H⁺ exchanger, for the purpose of diagnosing hypertension and proliferative diseases.

16. The use of a compound I as claimed in claim 1 for preparing a medicament for the treatment or prophylaxis of disturbances of fat metabolism.

17. A pharmaceutical which comprises an effective quantity of a compound I as claimed in one or more of claims 1 to 3.

## Revendications

1. Benzoylguanidines de formule I 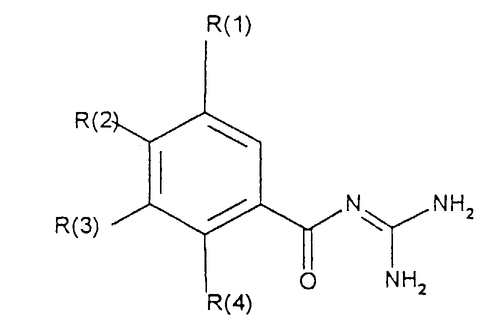 dans laquelle
R(1) représente un groupe R(13)-SOₘ ou R(14)R(15)N-SO₂-,
m est 1 ou 2,
R(13) représente un groupe alkyle ayant 1, 2, 3, 4, 5, 6, 7 ou 8 atomes de carbone, perfluoroalkyle ayant 1, 2, 3, 4, 5, 6, 7 ou 8 atomes de carbone, alcényle ayant 3, 4, 5, 6, 7 ou 8 atomes de carbone ou -CₙH₂ₙ-R(16),
n est égal à zéro, 1, 2, 3 ou 4,
R(16) représente un groupe cycloalkyle ayant 3, 4, 5, 6, 7 ou 8 atomes de carbone, phényle, biphénylyle ou naphtyle,
les radicaux phényle, biphénylyle et naphtyle n'étant pas substitués ou portant 1 à 3 substituants choisis dans l'ensemble constitué par F, Cl, CF₃, les groupes méthyle, méthoxy et NR(25)R(26),
R(25) et R(26) représentant,
indépendamment l'un de l'autre, un atome d'hydrogène ou un groupe alkyle ayant 1, 2, 3 ou 4 atomes de carbone ou perfluoroalkyle ayant 1, 2, 3 ou 4 atomes de carbone,
R(14) représente un atome d'hydrogène ou un groupe alkyle ayant 1, 2, 3, 4, 5, 6, 7 ou 8 atomes de carbone, perfluoroalkyle ayant 1, 2, 3, 4, 5, 6, 7 ou 8 atomes de carbone, alcényle ayant 3, 4, 5, 6, 7 ou 8 atomes de carbone ou -CₙH₂ₙ-R(27),
n est égal à zéro, 1, 2, 3 ou 4,
R(27) représente un groupe cycloalkyle ayant 3, 4, 5, 6, 7 ou 8 atomes de carbone, phényle, biphénylyle ou naphtyle,
les radicaux phényle, biphénylyle et naphtyle n'étant pas substitués ou portant 1 à 3 substituants choisis dans l'ensemble constitué par F, Cl, CF₃, les groupes méthyle, méthoxy et NR(28)R(29),
R(28) et R(29) représentant, indépendamment l'un de l'autre, un atome d'hydrogène ou un groupe alkyle ayant 1, 2, 3 ou 4 atomes de carbone ou perfluoroalkyle ayant 1, 2, 3 ou 4 atomes de carbone,
R(15) représente un atome d'hydrogène ou un groupe alkyle ayant 1, 2, 3 ou 4 atomes de carbone ou perfluoroalkyle ayant 1, 2, 3 ou 4 atomes de carbone,
ou
R(14) et R(15) représentent ensemble 4 ou 5 groupes méthylène, dont un groupe CH₂ peut être remplacé par un atome d'oxygène ou de soufre ou par NH, N-CH₃ ou par le groupe N-benzyle,
l'un des substituants R(2) et R(3) représente un atome d'hydrogène,
et l'autre substituant R(2) ou R(3) représente
-CHR(30)R(31),
R(30) représente
-(CH₂)_{g}-(CHOH)ₕ-(CH₂)ᵢ-(CHOH)ₖ-R(32) ou
-(CH₂)_{g}-O-(CH₂-CH₂O)ₕ-R(24)
R(24) et R(32)
représentant, indépendamment l'un de l'autre, un atome d'hydrogène ou le groupe méthyle,
g, h, i
étant égaux ou différents et représentant zéro, 1, 2, 3 ou 4,
k étant 1, 2, 3 ou 4,
ou l'autre substituant R(2) ou R(3) représente
-C(OH)R(33)R(34),
R(31), R(33) et R(34)
sont identiques ou différents et représentent un atome d'hydrogène ou un groupe alkyle ayant 1, 2, 3 ou 4 atomes de carbone,
ou
R(33) et R(34)
forment ensemble un groupe cycloalkyle ayant 3, 4, 5 ou 6 atomes de carbone,
ou
R(33) représente -CH₂OH,
R(4) représente un groupe alkyle ayant 1, 2, 3 ou 4 atomes de carbone, alcoxy ayant 1, 2, 3 ou 4 atomes de carbone, F, Cl, Br, I, CN, -(CH₂)ₙ-(CF₂)ₒ-CF₃,
n est zéro ou 1,
o est zéro, 1 ou 2,
et leurs sels pharmaceutiquement acceptables.

2. Composé de formule I selon la revendication 1, dans lequel
R(1) représente un groupe R(13)-SO₂ ou R(14)R(15)N-SO₂-,
R(13) représente un groupe alkyle ayant 1, 2, 3 ou 4 atomes de carbone, perfluoroalkyle ayant 1, 2, 3 ou 4 atomes de carbone, alcényle ayant 3 ou 4 atomes de carbone ou -CₙH₂ₙ-R(16),
n est égal à zéro, 1, 2, 3 ou 4,
R(16) représente un groupe cycloalkyle ayant 3, 4, 5 ou 6 atomes de carbone, phényle, biphénylyle ou naphtyle,
les radicaux phényle, biphénylyle et naphtyle n'étant pas substitués ou portant 1 à 3 substituants choisis dans l'ensemble constitué par F, Cl, CF₃, les groupes méthyle et méthoxy,
R(14) représente un atome d'hydrogène ou un groupe alkyle ayant 1, 2, 3 ou 4 atomes de carbone, perfluoroalkyle ayant 1, 2, 3 ou 4 atomes de carbone, alcényle ayant 3 ou 4 atomes de carbone ou -CₙH₂ₙ-R(27),
n est égal à zéro, 1, 2, 3 ou 4,
R(27) représente un groupe cycloalkyle ayant 3, 4, 5, 6, 7 ou 8 atomes de carbone, phényle, biphénylyle ou naphtyle,
les radicaux phényle, biphénylyle et naphtyle n'étant pas substitués ou portant 1 à 3 substituants choisis dans l'ensemble constitué par F, Cl, CF₃, les groupes méthyle et méthoxy,
R(15) représente un atome d'hydrogène ou un groupe alkyle ayant 1, 2, 3 ou 4 atomes de carbone ou perfluoroalkyle ayant 1, 2, 3 ou 4 atomes de carbone,
ou
R(14) et R(15) représentent ensemble 4 ou 5 groupes méthylène, dont un groupe CH₂ peut être remplacé par un atome d'oxygène ou de soufre ou par NH, N-CH₃ ou par le groupe N-benzyle,
l'un des substituants R(2) et R(3) représente un atome d'hydrogène,
et l'autre substituant R(2) ou R(3) représente
-CHR(30)R(31),
R(30) représente
- (CH₂)_{g}-(CHOH)ₕ-(CH₂)ᵢ-(CHOH)ₖ-R(32) ou
-(CH₂)_{g}-O-(CH₂-CH₂O)ₕ-R(24)
R(24) et R(32)
représentant, indépendamment l'un de l'autre, un atome d'hydrogène ou le groupe méthyle,
g, h, i
étant égaux ou différents et représentant zéro, 1 ou 2,
k étant 1 ou 2,
ou l'autre substituant R(2) ou R(3) représente
- C(OH)R(33)R(34),
R(33) et R(34)
sont identiques ou différents et représentent un atome d'hydrogène ou un groupe alkyle ayant 1, 2, 3 ou 4 atomes de carbone,
ou
R(33) et R(34)
forment ensemble un groupe cycloalkyle ayant 3, 4, 5 ou 6 atomes de carbone,
ou
R(33) représente -CH₂OH,
R(4) représente un groupe alkyle ayant 1, 2, 3 ou 4 atomes de carbone, alcoxy ayant 1, 2, 3 ou 4 atomes de carbone, F' Cl, CN ou (CF₂)ₒ-CF₃,
o est zéro, 1 ou 2.

3. Composé de formule I selon la revendication 1 ou 2, dans lequel
R(1) représente un groupe R(13)-SO₂,
R(13) représente un groupe alkyle ayant 1, 2, 3 ou 4 atomes de carbone,
l'un des substituants R(2) et R(3) représente un atome d'hydrogène,
et l'autre substituant R(2) ou R(3) représente
-C(OH)(CH₃)-CH₂OH, -CH(CH₃)-CH₂OH ou C(OH)(CH₃)₂,
R(4) représente un groupe alkyle ayant 1, 2, 3 ou 4 atomes de carbone, alcoxy ayant 1, 2, 3 ou 4 atomes de carbone, F, Cl, CN ou -CF₃.

4. Procédé pour la préparation d'un composé I selon la revendication 1, caractérisé en ce que l'on fait réagir avec de la guanidine un composé de formule II 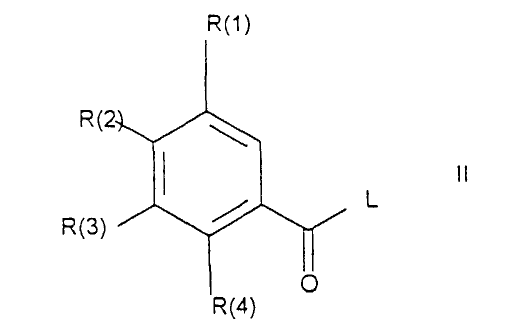 dans laquelle R(1) à R(4) ont les significations données dans la revendication 1 et L représente un groupe partant pouvant être aisément remplacé par substitution nucléophile.

5. Utilisation d'un composé I selon la revendication 1, pour la fabrication d'un médicament destiné au traitement et à la prophylaxie de maladies provoquées par des états ischémiques.

6. Utilisation d'un composé I selon la revendication 1, pour la fabrication d'un médicament destiné au traitement ou à la prophylaxie de l'infarctus du myocarde et d'arythmies.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Utilisation d'un composé I selon la revendication 1, pour la fabrication d'un outil scientifique destiné à l'inbition de l'échangeur Na⁺/H⁺, pour le diagnostic de l'hypertonie et de maladies proliférantes.

16. Utilisation d'un composé I selon la revendication 1, pour la fabrication d'un médicament destiné au traitement et à la prophylaxie de troubles du métabolisme des lipides.

17. Médicament, contenant une quantité efficace d'un composé I selon une ou plusieurs des revendications 1 à 3.
